(19) 

Europäisches Patentamt
European Patent Office
Office européen des brevets

(11) **EP 4 548 833 A1**

(12) **EUROPEAN PATENT APPLICATION**
published in accordance with Art. 153(4) EPC

(43) Date of publication:
07.05.2025 Bulletin 2025/19

(21) Application number: 23831868.7

(22) Date of filing: 27.06.2023

(51) International Patent Classification (IPC):
*A61B 5/00* (2006.01)

(52) Cooperative Patent Classification (CPC):
A61B 5/00

(86) International application number:
PCT/KR2023/008955

(87) International publication number:
WO 2024/005509 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.06.2022 KR 20220079124

(71) Applicant: Lululab Inc.
Seoul 06054 (KR)

(72) Inventors:
• JUNG, Geunho
 Seoul 06041 (KR)
• LEE, Jong Ha
 Hwaseong-si, Gyeonggi-do 18456 (KR)
• YOO, Sangwook
 Seoul 07986 (KR)
• CHOE, Yongjoon
 Seoul 06305 (KR)

(74) Representative: Franke, Dirk
Franke & Partner
Patent- und Rechtsanwälte
Widenmayerstraße 25
80538 München (DE)

(54) **METHOD AND DEVICE FOR ACQUIRING SKIN ANALYSIS INFORMATION USING MULTIPLE WAVELENGTHS**

(57) According to various embodiments, a multi-wavelength skin analysis device comprises: a skin condition measuring unit for measuring the user's skin condition using a camera and a multi-wavelength light source; a multi-purpose skin analysis information acquisition unit for acquiring the user's skin analysis information through multi-wavelength images acquired from the multi-wavelength light source; a solution providing unit for offering information on recommended cosmetics corresponding to the measured skin condition, wherein the multi-purpose skin analysis information acquisition unit may include a melanin distribution confirmation unit for determining the melanin state of the user's skin, a dark circle cause determination unit for identifying the cause of dark circles, and a wrinkle cause determination unit corresponding to the differentiation of wrinkle-forming layers.

FIG. 1

10

(200)
**User terminal**

**Multi-wavelength skin analysis device (100)**

EP 4 548 833 A1

## Description

## Technical Field

The present disclosure relates to a method and device for acquiring skin analysis information using multiple wavelengths and, more specifically, to a method and device for acquiring multi-purpose skin analysis information using multiple wavelengths, which enables determination of the cause of skin melanin distribution, identification of dark circles, and identification of a wrinkle-forming layer.

## Background Art

Unless otherwise indicated herein, the contents described in this section are not prior art to the claims of this disclosure, and their inclusion in this section is not deemed to be prior art.

Recently, interest in skin health, especially in facial skin health, has been increasing. As interest in skin health has increased, various cosmetics and beauty devices have been introduced. Further, there is developed a skin condition measuring method that takes a picture of a user's facial skin and then analyzes various troubles (e.g., wrinkles, pores, acne, etc.) on the user's facial skin.

According to the existing skin condition measuring method for skin analysis, the user's face is photographed based on predetermined photographing parameters (e.g., light source intensity, direction, aperture value, shutter speed, etc.) through a skin condition measuring device or apparatus, and then the user's skin condition is measured using images of the photographed user's face.

However, since such a skin condition measuring method performed using the user's facial images uses parameters that are insufficient for diagnosing the skin, it diagnoses only the superficial skin condition and cannot find the fundamental cause of the trouble. For example, when diagnosing the user's wrinkles with a system for analyzing 2D images that use only white light or UV wavelengths as a light source, fine wrinkles on the epidermis and deep wrinkles created in the dermis are mixed and thus it is difficult to distinguish between them.

In addition, it may be difficult to analyze the skin variously in only a single measurement using the above-described method. Therefore, there is a need for a method to acquire multi-purpose skin analysis information using a multi-wavelength light source when diagnosing the skin.

## Disclosure

## Technical Problem

Accordingly, the present disclosure has been made keeping in mind the above problems occurring in the related art, and an objective of the present disclosure is to provide a method and device for acquiring multi-purpose skin analysis information using multiple wavelengths, by utilizing the characteristics of changes in absorption and scattering within the skin according to the length of the wavelength and the difference in light penetration depth.

## Technical Solution

According to various embodiments, a multi-wavelength skin analysis device may include a skin condition measuring unit measuring a user's skin condition using a camera and a multi-wavelength light source; a multi-purpose skin analysis information acquisition unit acquiring the user's skin analysis information through multi-wavelength images acquired from the multi-wavelength light source; and a solution providing unit offering information on recommended cosmetics corresponding to the measured skin condition.

According to various embodiments, the multi-purpose skin analysis information acquisition unit may include a melanin distribution confirmation unit for determining a melanin state of the user's skin, a dark circle cause determination unit for determining a cause of dark circles, and a wrinkle cause determination unit for determining a cause of wrinkle based on identification of wrinkle-forming layers.

According to various embodiments, the melanin distribution confirmation unit may acquire a first wavelength image using a first light source having a first wavelength, acquire a second wavelength image using a second light source having a second wavelength that is shorter than the first wavelength, extracts a first melanin region image based on the first wavelength image, extract a second melanin region image based on the second wavelength image, and combine the first melanin region image and the second melanin region image with a skin image of the user to acquire a melanin distribution image through the combination. The method may include repeating the wavelength image acquisition and melanin region image extraction operations using two or more different wavelength light sources in the same manner, and obtaining a melanin multi-stage distribution image corresponding to the melanin light absorption characteristics of each wavelength through the above-described combination.

According to various embodiments, the melanin distribution confirmation unit may acquire a third wavelength image using a third light source having a third wavelength, which is a longest wavelength among the multi-wavelength light source, acquire a fourth wavelength image using a fourth light source having a fourth wavelength, which is a shortest wavelength among the multi-wavelength light source, generate a differential image based on the third wavelength image and the fourth wavelength image, and acquire a distribution image in which a melanin region is emphasized based on the generated differential image.

According to various embodiments, the dark circle cause determination unit may detect a dark circle area of the user from a skin image of the user acquired through the camera, determine that a cause of a user's dark circle is caused due to pigmentation when a melanin component is detected above a predetermined first component amount in the detected dark circle area, and determine that the cause of the user's dark circle is caused due to vasodilation when a hemoglobin component is detected above a predetermined second component amount in the detected dark circle area.

According to various embodiments, the wrinkle cause determination unit may acquire a first wrinkle image based on a fifth light source having a fifth wavelength among the multi-wavelengths, acquire a second wrinkle image based on a sixth light source having a sixth wavelength that is longer than the fifth wavelength among the multi-wavelengths, and determine wrinkle classification depending on a wrinkle creation layer of the user and a main cause of wrinkle creation corresponding to the wrinkle classification, based on the first wrinkle image and the second wrinkle image, in which the first wrinkle image is an epidermal wrinkle image, and the second wrinkle information is a dermal wrinkle image.

**Advantageous Effects**

According to various embodiments disclosed herein, multi-purpose skin analysis information can be acquired simultaneously using multi-wavelengths.

In addition, according to various embodiments, the melanin distribution in the user's skin can be confirmed using a multi-wavelength light source.

In addition, according to various embodiments, the cause of dark circles can be identified using a multi-wavelength light source, and a dark circle solution method can be suggested according to the cause thereof.

In addition, according to various embodiments, it is possible to distinguish between shallow wrinkles of the epidermis and deep wrinkles created in the dermis using a multi-wavelength light source and suggest a user-customized solution method according to the classification of the wrinkle creation layer.

In addition, various effects that are directly or indirectly identified through this disclosure can be provided.

**Description of Drawings**

FIG. 1 is a drawing illustrating multi-wavelength skin analysis information according to an embodiment.
FIG. 2 is a drawing illustrating the main components of a multi-wavelength skin analysis device.
FIG. 3 is a flow diagram illustrating a method of confirming the melanin distribution of a user's skin through a multi-wavelength light source.
FIG. 4 is a flow diagram illustrating a method of determining the cause of dark circles of a user using a multi-wavelength light source.
FIG. 5 is a flow diagram illustrating a method of determining the cause of wrinkle creation based on the classification of wrinkle formation layers of a user's skin through a multi-wavelength light source.
FIG. 6 is a drawing illustrating the hardware configuration of a multi-wavelength skin analysis device according to FIG. 1.

**Mode for Invention**

The present disclosure can have various modifications and various embodiments, and specific embodiments will be illustrated in the drawings and described herein in detail. However, it should be appreciated that the description is not intended to limit the present disclosure to specific embodiments, and includes all modifications, equivalents, or substitutes included in the spirit and technical scope of the present disclosure. When describing each drawing, similar reference numerals are used for similar components.

The terms "first", "second", "A", "B", etc. may be used to describe various components, but the components should not be limited by the terms. The terms are used only for the purpose of distinguishing one component from another. For example, without departing from the scope of the present disclosure, the first component may be referred to as the second component, and similarly, the second component may also be referred to as the first component. The term "and/or" includes a combination of a plurality of related described items or any of a plurality of related described items.

It will be understood that when an element is referred to as being "connected to" or "coupled to" another element, it can be directly connected or coupled to the other element or intervening elements may be present. In contrast, it will be understood that when an element is referred to as being "directly connected to" or "directly coupled to" another element, there are no intervening elements.

The terms used herein are used only to describe specific embodiments and are not intended to limit the present disclosure. The singular terms may include the plural term unless the context clearly indicates otherwise. In this disclosure, the terms "comprise" or "have" should be understood to indicate the presence of a feature, number, step, operation, component, part, or combination thereof described in the specification, but do not preclude the possibility of the presence or addition of one or more other features, numbers, steps, operations, components, parts, or combinations thereof.

Unless otherwise defined, all terms used herein, including technical or scientific terms, have the same meaning as commonly understood by a person of ordinary skill in the art to which the present disclosure belongs. Further, it will be understood that terms, such as those defined in commonly used dictionaries, should be interpreted as having a meaning that is consistent with their meaning in the context of the relevant art and will not be interpreted in an idealized or overly formal sense unless explicitly so defined herein.

Hereinafter, preferred embodiments according to the present disclosure will be described in detail with reference to the attached drawings.

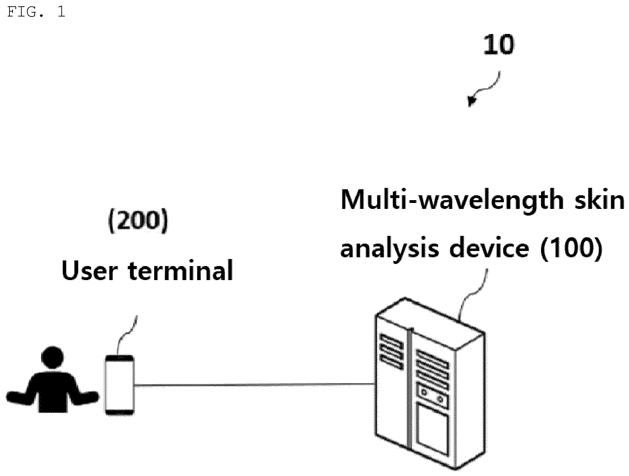

FIG. 1 is a diagram illustrating multi-wavelength skin analysis information 10 according to an embodiment. Referring to FIG. 1, multi-wavelength skin analysis information 10 may include a multi-wavelength skin analysis device 100, a user terminal 200, and the like.

The multi-wavelength skin analysis device 100 may include a multi-wavelength light source (e.g., an ultraviolet light source, a visible light source, a near-infrared light source) and a photographing unit (e.g., a camera), and may measure the user's skin condition through the multi-wavelength light source and the photographing unit. The multi-wavelength skin analysis device 100 may extract the melanin distribution, determine the cause of the user's dark circles, and determine the cause of the user's wrinkles, based on a multi-wavelength image acquired through the multi-wavelength light source and a user's face image acquired through the photographing unit.

The user terminal 200 may be a desktop computer, a laptop computer, a notebook, a smart phone, a tablet PC, a mobile phone, a smart watch, a smart glass, an e-book reader, a portable multimedia player (PMP), a portable game console, a navigation device, a digital camera, a digital multimedia broadcasting (DMB) player, a digital audio recorder, a digital audio player, a digital video recorder, a digital video player, a Personal Digital Assistant (PDA), etc.

The multi-wavelength skin analysis device 100 and the user terminal 200 are each connected to a communication network to transmit and receive data to each other through the communication network. For example, the communication network may include various types of wired or wireless networks, such as Local Area Network (LAN), Metropolitan Area Network (MAN), Global System for Mobile Network (GSM), Enhanced Data GSM Environment (EDGE), High Speed Downlink Packet Access (HSDPA), Wideband Code Division Multiple Access (W-CDMA), Code Division Multiple Access (CDMA), Time Division Multiple Access (TDMA), Bluetooth, Zigbee, Wi-Fi, Voice over Internet Protocol (VoIP), LTE Advanced, IEEE802.16m, WirelessMAN-Advanced, HSPA+, 3GPP Long Term Evolution (LTE), Mobile WiMAX (IEEE 802.16e), UMB (formerly EV-DO Rev.C), Flash-OFDM, iBurst and MBWA (IEEE 802.20) systems, HIPERMAN, Beam-Division Multiple Access (BDMA), World Interoperability for Microwave Access (Wi-MAX), 5G, etc.

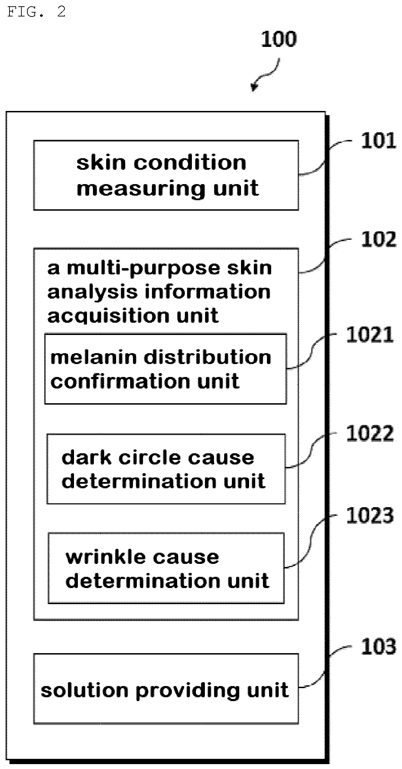

FIG. 2 is a drawing showing main components of a multi-wavelength skin analysis device 100. Referring to FIG. 2, the multi-wavelength skin analysis device 100 may include a skin condition measuring unit 101, a multi-purpose skin analysis information acquisition unit 102, a melanin distribution confirmation unit 1021, a dark circle cause determination unit 1022, a wrinkle cause determination unit 1023, and a solution providing unit 103. It will be appreciated by those skilled in the art that the multi-purpose skin analysis information acquisition unit 102 is not limited to including the melanin distribution confirmation unit 1021, the dark circle cause determination unit 1022, and a wrinkle cause determination unit 1023, and it may further include other analysis units that are capable of performing analysis using multiple wavelengths.

The skin condition measuring unit 101 may include an ultraviolet light source unit that irradiates ultraviolet rays, a visible light source unit that irradiates visible light, and an infrared light source unit that irradiates near-infrared rays. In addition to the multi-wavelength light source, the skin condition measuring unit 101 may acquire an image photographing unit that includes a camera, a polarizing plate, a diffusion plate, a distance detection sensor, etc.

The skin condition measuring unit 101 may irradiate the user's skin with a multi-wavelength light source including ultraviolet (UV), visible light (VIS), and near-infrared (NIR) light sources. The skin condition measuring unit 101 may photograph the user's skin through the photographing unit, while irradiating it with the multi-wavelength light source. The skin condition measuring unit 101 may acquire the user's skin image by photographing it.

The skin condition measuring unit 101 may sequentially emit multiple light sources such as ultraviolet rays, visible light, and infrared rays, acquire wavelength band images corresponding to each of the multiple light sources through a camera, and include a wavelength band filter in the light source or the camera.

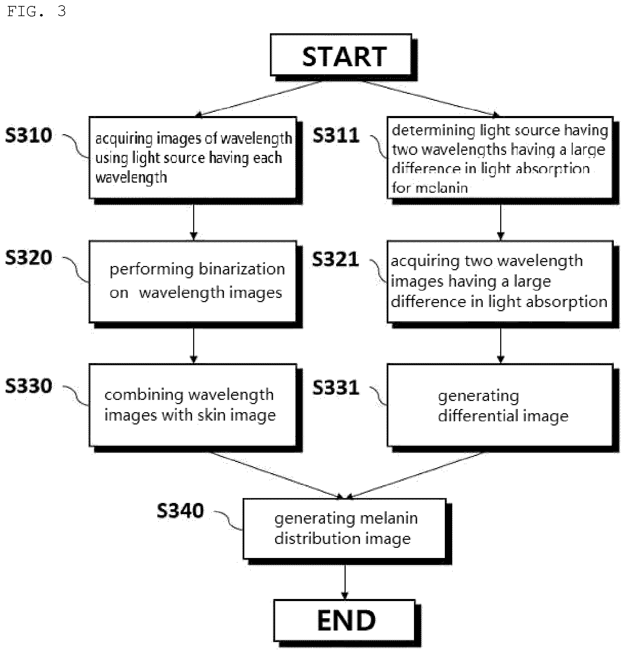

FIG. 3 is a flow diagram illustrating a method of confirming the melanin distribution of a user's skin using a multi-wavelength light source.

The melanin distribution confirmation unit 1021 may confirm the melanin distribution through the multi-wavelength light source. The melanin distribution confirmation unit 1021 may acquire the melanin distribution image based on the melanin absorbance characteristics depending on wavelength through a multi-wavelength light source and calculate the melanin index.

The melanin distribution confirmation unit 1021 may acquire images of each wavelength using a light source having each wavelength (S310), in which even areas with low melanin density in the image show high contrast with the surrounding skin tissue due to the high melanin absorbance at relatively short wavelengths (e.g., visible light and UV-A below 450 nm), while only areas with high melanin density in the image show high contrast with the surrounding skin tissue due to the low melanin absorbance at relatively long wavelengths (e.g., visible light and near-infrared above 750 nm).

The melanin distribution confirmation unit 1021 may perform binarization (e.g., Otsu's Thresholding) on each of the wavelength images (S320). The melanin distribution confirmation unit 1021 may extract melanin regions of each of the wavelength images for which binarization has been performed. The melanin distribution confirmation unit 1021 may combine the image of the extracted melanin regions with the user's skin image (S330). The melanin distribution confirmation unit 1021 may acquire a melanin distribution image through the combination (S340).

In other words, the melanin distribution confirmation unit 1021 may acquire a first wavelength image using a first light source having a first wavelength, and a second wavelength image using a second light source having a second wavelength that is shorter than the first wavelength. The melanin distribution confirmation unit 1021 may extract a first melanin region based on the first wavelength image and a second melanin region based on the second wavelength image. The melanin distribution confirmation unit 1021 may acquire a melanin distribution image by combining the first melanin region image and the second melanin region image with the skin image of the user. The melanin distribution confirmation unit 1021 may acquire multi-stage melanin distribution images corresponding to the melanin light absorption characteristics for each wavelength through the combination, by repeating operations of acquiring the wavelength image and extracting melanin area image using two or more different wavelength light sources.

The melanin distribution confirmation unit 1021 may determine a light source having two wavelengths each having a large difference in light absorption for melanin (S311). The melanin distribution confirmation unit 1021 may acquire two wavelength images (e.g., a first wavelength image and a second wavelength image) having a large difference in light absorption for melanin (S321). The melanin distribution confirmation unit 1021 may generate a differential image based on the first wavelength image and the second wavelength image (S331).

In other words, the melanin distribution confirmation unit 1021 may acquire a third wavelength image using a third light source having a third wavelength, which is the longest wavelength among the multi-wavelength light sources and acquire a fourth wavelength image using a fourth light source having a fourth wavelength, which is the shortest wavelength among the multi-wavelength light sources. The melanin distribution confirmation unit 1021 may generate a differential image based on the third wavelength image and the fourth wavelength image. The melanin distribution confirmation unit 1021 may generate a melanin distribution image (e.g., a melanin-enhanced image) based on the differential image (S340).

The melanin distribution confirmation unit 1021 may calculate a ratio of the pixel value included in the melanin area to the light source intensity before light absorption and calculate the melanin level (melanin index) based on the calculated ratio.

In other words, when a ratio (diffuse reflectance) of the light source to the detected light diffused and reflected inside the skin is termed 'R', the optical density (OD) is as follows, in which the melanin index image may be acquired in such a way to calculate the optical density gradient between two wavelengths by multiplying the optical density difference of the pixels corresponding to the same location in the two wavelength images with different light absorbances by a compensation constant, and the melanin level may be acquired by calculating the index average value of the entire image or the melanin region.

[Equation 1]

$$OD = -\log R$$

In Equation 1, OD may represent an optical density, and R may represent a ratio of the emitted light to the detected light diffused and reflected inside the skin.

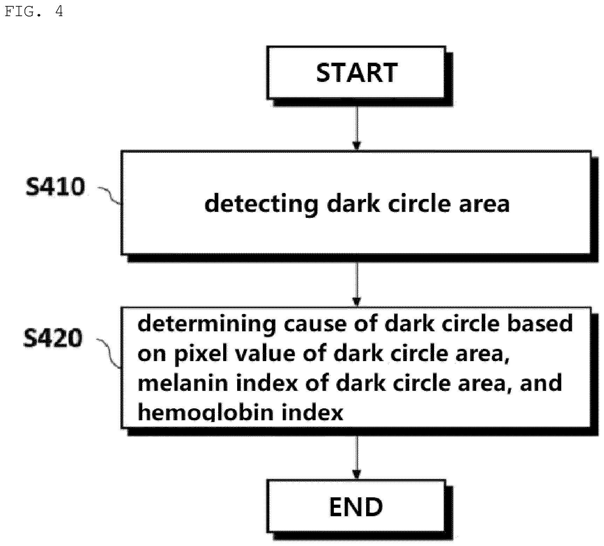

FIG. 4 is a flow diagram illustrating a method of determining the cause of dark circles of a user using a multi-wavelength light source.

The dark circle cause determination unit 1022 measures melanin and hemoglobin components through multiple wavelengths and determines the cause of dark circles based on the measured melanin and hemoglobin components.

The dark circle cause determination unit 1022 may detect an eye area in a user's face image. The dark circle cause determination unit 1022 may detect a dark circle area close to the eye area in the user's face image. The dark circle cause determination unit 1022 may select a dark circle area more restrictively, and determine a dark circle expected area in order to detect a dark circle within the restricted area. The dark circle cause determination unit 1022 may detect a left eye and a right eye within an acquired face image, determine the outermost lines of each of the detected left and right eyes, and determine a point that is bent by a predetermined angle or more from the determined outermost lines. For example, the dark circle cause determination unit 1022 may determine the first point and the second point that are bent by the predetermined angle or more from the first outermost line corresponding to the left eye, and the third point and the fourth point that are bent by the predetermined angle or more from the second outermost line corresponding to the right eye.

The dark circle cause determination unit 1022 may calculate a first distance between the first point and the second point, and a second distance between the third point and the fourth point. The dark circle cause determination unit 1022 may determine a first area from the first lowest point of the first outermost line corresponding to the left eye to the point that is the first distance downward, and a second area from the second lowest point of the second outermost line corresponding to the right eye to the point that is the second distance downward, as the dark circle expected area, respectively.

The dark circle cause determination unit 1022 may train a dark circle area detection model, which is an artificial neural network model, and input a user's face image into the trained dark circle area detection model. The dark circle cause determination unit 1022 may detect or determine the dark circle area by inputting the user's face image into the model (S410).

The dark circle cause determination unit 1022 may use facial images of people with dark circles as learning data. The facial images of people with dark circles may be acquired from a database included in the device 100 or terminal 200, or acquired from an external DB. The learning data may be training data which have feature points of the facial image as input values and feature points of the dark circle area in the facial image as output values. The feature points may be pixel values or color values corresponding to each pixel.

The dark circle cause determination unit 1022 may learn the dark circle area detection model using learning data. The dark circle area detection model may be supervised learning using 'feature points of the face image' and 'feature points of the dark circle area in the face image'. The supervised learning refer to learning that finds an output value according to a given input value by using data with input values and corresponding output values as learning data, in which the learning is performed in a state where the correct answer is known. The set of input values and output values given to the supervised learning is referred to as training data. That is, the 'feature points of the face image' and the 'feature points of the dark circle area in the face image' described above may be input values and output values, respectively, which are used as training data for supervised learning of the dark circle area detection model.

For example, the dark circle cause determination unit 1022 may convert the feature points of the face image into a unique first one-hot vector to generate an input value, and convert the feature points of the dark circle area in the face image into a unique second one-hot vector to generate an output value, and then perform supervised learning on the dark circle area detection model using the generated input and output values. Here, the first one-hot vector and the second one-hot vector may be vectors in which one of the component values constituting the vector is '1' and the remaining component values are '0'.

In one embodiment, the dark circle area detection model may include an input layer that receives input values and has nodes corresponding to the number of components of the first one-hot vector; one or more hidden layers that multiply each output value of the input layer by a connection strength (or weight) and add a bias to it thereby to output the result; and an output layer that multiplies each output value of the hidden layer by a connection strength (or weight) thereby to output the result using an activation function. For example, the activation function may be, but is not limited to, a ReLU function or a Softmax function. The connection strength and the bias may be continuously updated by supervised learning.

Specifically, the supervised learning may be performed on the dark circle area detection model so that the output value of the loss function according to the given input value (the first one-hot vector) and output value (the second one-hot vector) is minimized. For example, the loss function (H(Y, Y')) may be defined as in Equation 2 below.

$$[Equation\ 2]$$

$$H(Y,Y') = -\sum_{m=1}^{N} Y_m \cdot log(Y'_m)$$

Herein, Ym may be the mth component of the second one-hot vector, and Y'm may be the mth component of the output vector output by receiving the first one-hot vector from the average face image estimation model 503.

When the dark circle cause determination unit 1022 determines the dark circle area based on the 'feature points of the dark circle area in the face image', which are output values of the dark circle area detection model, it may determine the dark circle area additionally considering the determined dark circle expected area. For example, the dark circle cause determination unit 1022 may determine the dark circle area based on the feature points that overlap with the dark circle expected area among the 'feature points of the dark circle area in the face image'.

The dark circle cause determination unit 1022 may calculate a first average value for pixel values of pixels included in a determined or detected dark circle area, and calculate a second average value for pixel values of pixels included in a non-dark circle area adjacent to the dark circle area.

The dark circle cause determination unit 1022 may determine that the user's dark circle is caused due to pigmentation, when the difference between the first average value and the second average value is greater than or equal to a predetermined difference value and the melanin index in the detected dark circle area is greater than or equal to a predetermined melanin threshold index.

The dark circle cause determination unit 1022 may determine that the user's dark circle is caused due to pigmentation, when the difference between the first average value and the second average value is greater than or equal to a predetermined difference value and the hemoglobin index in the detected dark circle area is greater than or equal to a predetermined hemoglobin threshold index.

In other words, since melanin among skin components is directly related to pigmentation and hemoglobin is directly related to vasodilation, the dark circle cause determination unit 1022 may determine that the user's dark circles is caused due to pigmentation when the melanin component is detected above the predetermined first component amount, and determine that the user's dark circles are caused due to vasodilation when the hemoglobin component is detected above the predetermined second component amount.

The dark circle cause determination unit 1022 may determine the melanin measurement wavelength and hemoglobin measurement wavelength based on the light absorbance characteristics. The dark circle cause determination unit 1022 may acquire each measurement wavelength light source image (e.g., melanin measurement wavelength light source image and hemoglobin measurement wavelength light source image) measured using a reflector, and a detection light image generated when each wavelength is diffused and reflected within skin tissue in the dark circle area, and calculate the optical density in pixels included in the dark circle area based on the intensity ratio of each pixel of the two images.

The melanin index image may be derived by a method of calculating the optical density gradient between two wavelengths by multiplying the optical density difference of pixels corresponding to the same location in two wavelength images with different optical absorbance for melanin by a compensation constant, which may include a method of using light sources with wavelengths of about 600 nm and 700 nm, particularly 650 nm and 700 nm. The hemoglobin index image may be derived by finding the optical density gradient between wavelengths of about 560 nm and 650 nm, and/or fining the optical density difference between two wavelength images with different optical absorbance for hemoglobin, in order to calculate the area between about 500 nm and 600 nm in a skin optical density graph.

The dark circle cause determination unit 1022 may generate a melanin index image and a hemoglobin index image through the calculation and determine or calculate the melanin index and the hemoglobin index based on the melanin index image and the hemoglobin index image.

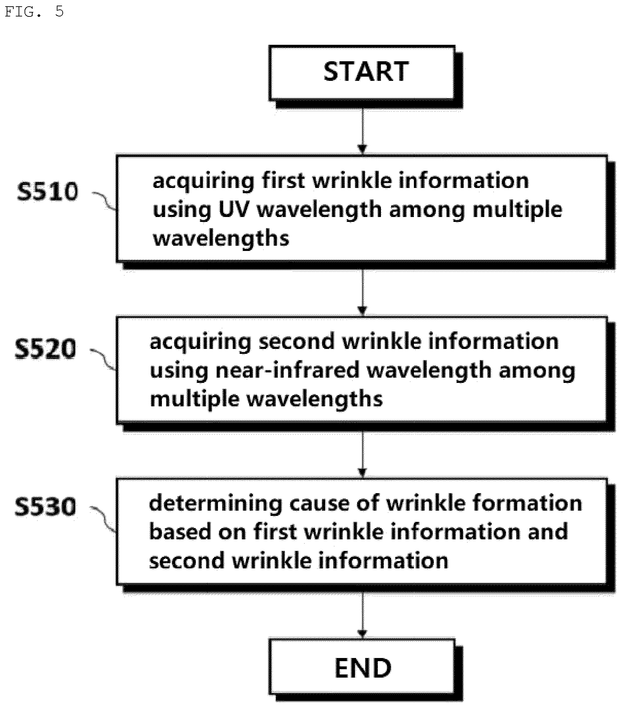

FIG. 5 is a flow diagram illustrating a method of determining the cause of wrinkles according to the classification of wrinkle formation layers of a user's skin using a multi-wavelength light source.

The wrinkle cause determination unit 1023 may acquire wrinkle information through multiple wavelengths. The wrinkle cause determination unit 1023 may acquire the first wrinkle information (e.g., shallow wrinkle emphasis information) using the fifth wavelength (e.g., UV wavelength) among the multiple wavelengths (S510), and acquire the second wrinkle information (e.g., deep wrinkle information) using the sixth wavelength (e.g., near-infrared wavelength) among the multiple wavelengths (S520). In other words, the wrinkle cause determination unit 1023 may determine the degree of fine wrinkles in the epidermis and deep wrinkles created in the dermis by using the characteristic of the difference in light penetration depth due to the change in absorption and scattering in skin tissue depending on the light wavelength, which is difficult to distinguish using the existing method using white light or UV wavelength light source. Due to the difference in light penetration depth, a mixed image of deep wrinkles with shallow wrinkles in the epidermis may be acquired at the fifth wavelength (e.g., UV wavelength), a mixed image of shallow and deep wrinkles may be acquired at the visible light source, and a deep wrinkle image in the dermis layer may be acquired at the sixth wavelength (e.g., near-infrared). A method of calculating the degree of wrinkles based on the acquired multi-wavelength skin image may include a wrinkle area emphasis method based on wrinkle detection model or image processing through training data which has wrinkle area feature points as output values.

The wrinkle cause determination unit 1023 may determine the cause of wrinkle formation in the user based on the acquired wrinkle information (S530).

The wrinkle cause determination unit 1023 may acquire a first wrinkle image of the user through a light source having a fifth wavelength and acquire a second wrinkle image of the user through a light source having a sixth wavelength that is longer than the fifth wavelength.

For example, the wrinkle cause determination unit 1023 determines that the user's wrinkles are shallow wrinkles that occurred in the epidermal layer, when the degree of the user's shallow wrinkles is greater than a predetermined degree based on shallow wrinkle information acquired based on the fifth wavelength (e.g., UV wavelength) or the difference between images of the fifth wavelength and the sixth wavelength. Based on the determination that the user's wrinkles are shallow wrinkles, the wrinkle cause determination unit 1023 may determine that the user's main wrinkle creation is caused due to deficiency of moisture.

For example, the wrinkle cause determination unit 1023 determines that the user's wrinkles are deep wrinkles that occurred in the dermis layer, when the user's deep wrinkle level is greater than a predetermined level based on deep wrinkle information acquired through the sixth wavelength (e.g., near-infrared). Based on the determination that the user's wrinkles are deep wrinkles, the wrinkle cause determination unit 1023 may determine that the user's main wrinkle creation is caused due to lack of elasticity.

The solution providing unit 103 may provide the user terminal 200 with information on recommended cosmetics or recommended products corresponding to the measured skin condition. When the main cause of wrinkles of the user is determined to be lack of moisture, the solution provider 103 may determine cosmetics or products with a good moisturizing effect as the cosmetics or products to recommend to the user and when the main cause of wrinkles of the user is determined to be lack of elasticity, the solution provider 103 may determine cosmetics or products with good skin elasticity as the cosmetics or products to recommend to the user. When the measured skin condition of the user is determined to have a high melanin index, the solution providing unit 103 may determine cosmetics or products with good whitening or pigmentation as the cosmetics or products to recommend to the user, and when the skin condition of the user is determined to have a high hemoglobin index, the solution provider 103 may determine cosmetics or products with good redness or vasodilation as the cosmetics or products to recommend to the user.

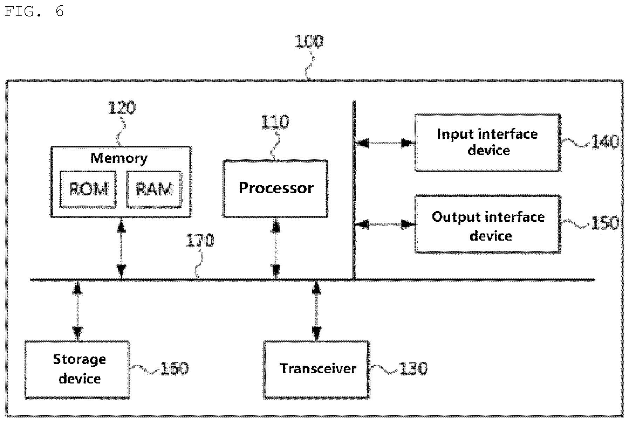

FIG. 6 is a drawing illustrating the hardware configuration of a multi-wavelength skin analysis device according to FIG. 1.

Referring to FIG. 6, the multi-wavelength skin analysis device 100 may include at least one processor 110 and a memory storing instructions that cause the at least one processor 110 to perform at least one operation.

The at least one operation may include at least some of the operations or functions of the above-described device 100 and may be implemented in the form of commands to be performed by the processor 110.

Here, at least one processor 110 may refer to a central processing unit (CPU), a graphics processing unit (GPU), or a dedicated processor on which methods are performed according to embodiments of the present disclosure. Each of the memory 120 and the storage device 160 may be configured with at least one of a volatile storage medium and a nonvolatile storage medium. For example, the memory 120 may be one of a read-only memory (ROM) and a random access memory (RAM), and the storage device 160 may be a flash memory, a hard disk drive (HDD), a solid state drive (SSD), or various memory cards (e.g., a micro SD card).

In addition, the multi-wavelength skin analysis device 100 may include a transceiver 130 that performs communication via a wireless network. In addition, the device 100 may further include an input interface device 140, an output interface device 150, a storage device 160, etc. Each component included in the multi-wavelength skin analysis device 100 may be connected by a bus 170 to communicate with each other. In FIG. 6, the multi-wavelength skin analysis device 100 is described as an example, but is not limited thereto. For example, a plurality of user terminals may include components according to FIG. 6.

The methods according to the present disclosure may be implemented in the form of program commands that can be executed through various computer means and recorded on a computer-readable medium. The computer-readable medium may include program commands, data files, data structures, etc., alone or in combination. The program commands recorded on the computer-readable medium may be those which are specifically designed and configured for the present disclosure, or which are known and available to those skilled in the art of computer software.

Examples of computer-readable media may include hardware devices specifically configured to store and execute program instructions, such as ROM, RAM, and flash memory. Examples of program instructions may include not only machine language codes, such as those produced by a compiler, but also high-level language codes that can be executed by a computer using an interpreter, etc. The hardware devices described above may be configured to operate with at least one software module to perform the operations of the present disclosure, and vice versa.

In addition, the above-described method or device may be implemented by combining all or part of its configuration or function or may be implemented separately.

Although the embodiments of the present disclosure have been described with reference to the above, it will be understood by those skilled in the art that various modifications and changes can be made to the present disclosure without departing from the spirit and scope of the present disclosure as set forth in the claims below.

**Claims**

1. A multi-wavelength skin analysis device, comprising:

   a skin condition measuring unit measuring a user's skin condition using a camera and a multi-wavelength light source;
   a multi-purpose skin analysis information acquisition unit acquiring the user's skin analysis information through multi-wavelength images acquired from the multi-wavelength light source; and
   a solution providing unit offering information on recommended cosmetics corresponding to the measured skin condition,
   wherein the multi-purpose skin analysis information acquisition unit comprises a melanin distribution confirmation unit for determining a melanin state of the user's skin, a dark circle cause determination unit for determining a cause of dark circles, and a wrinkle cause determination unit for determining a cause of wrinkle based on identification of wrinkle-forming layers.

2. The multi-wavelength skin analysis device of claim 1, wherein the melanin distribution confirmation unit,

   acquires a first wavelength image using a first light source having a first wavelength,
   acquires a second wavelength image using a second light source having a second wavelength that is shorter than the first wavelength,

extracts a first melanin region image based on the first wavelength image,

extracts a second melanin region image based on the second wavelength image, and

combines the first melanin region image and the second melanin region image with a skin image of the user to acquire a melanin distribution image through the combination.

3. The multi-wavelength skin analysis device of claim **1,** wherein the melanin distribution confirmation unit,

acquires a third wavelength image using a third light source having a third wavelength, which is a longest wavelength among the multi-wavelength light source,

acquires a fourth wavelength image using a fourth light source having a fourth wavelength, which is a shortest wavelength among the multi-wavelength light source,

generates a differential image based on the third wavelength image and the fourth wavelength image, and

acquires a distribution image in which a melanin region is emphasized based on the generated differential image.

4. The multi-wavelength skin analysis device of claim 1, wherein the dark circle cause determination unit,

detects a dark circle area of the user from a skin image of the user acquired through the camera,

determines that a cause of a user's dark circle is caused due to pigmentation when a melanin component is detected above a predetermined first component amount in the detected dark circle area, and

determines that the cause of the user's dark circle is caused due to vasodilation when a hemoglobin component is detected above a predetermined second component amount in the detected dark circle area.

5. The multi-wavelength skin analysis device of claim 1, wherein the wrinkle cause determination unit,

acquires a first wrinkle image based on a fifth light source having a fifth wavelength among the multi-wavelengths,

acquires a second wrinkle image based on a sixth light source having a sixth wavelength that is longer than the fifth wavelength among the multi-wavelengths, and

determines wrinkle classification depending on a wrinkle creation layer of the user and a main cause of wrinkle creation corresponding to the wrinkle classification, based on the first wrinkle image and the second wrinkle image,

wherein the first wrinkle image is an epidermal wrinkle image, and the second wrinkle information is a dermal wrinkle image.

FIG. 1

**10**

**Multi-wavelength skin**

**analysis device (100)**

**(200)**

**User terminal**

FIG. 2

FIG. 3

**START**

S310 acquiring images of wavelength using light source having each wavelength

S311 determining light source having two wavelengths having a large difference in light absorption for melanin

S320 performing binarization on wavelength images

S321 acquiring two wavelength images having a large difference in light absorption

S330 combining wavelength images with skin image

S331 generating differential image

S340 generating melanin distribution image

**END**

FIG. 4

```
        ┌─────────────────┐
        │      START      │
        └─────────────────┘
                 │
                 ▼
S410  ┌──────────────────────────────────────┐
      │       detecting dark circle area       │
      └──────────────────────────────────────┘
                 │
                 ▼
S420  ┌──────────────────────────────────────┐
      │ determining cause of dark circle based │
      │ on pixel value of dark circle area,    │
      │ melanin index of dark circle area, and │
      │ hemoglobin index                       │
      └──────────────────────────────────────┘
                 │
                 ▼
        ┌─────────────────┐
        │       END       │
        └─────────────────┘
```

FIG. 5

```
                              ┌──────────────┐
                              │    START     │
                              └──────┬───────┘
                                     │
                                     ▼
          ┌──────────────────────────────────────────────┐
  S510    │  acquiring first wrinkle information          │
          │  using UV wavelength among multiple          │
          │  wavelengths                                  │
          └──────────────────────┬───────────────────────┘
                                 │
                                 ▼
          ┌──────────────────────────────────────────────┐
  S520    │  acquiring second wrinkle information         │
          │  using near-infrared wavelength among        │
          │  multiple wavelengths                         │
          └──────────────────────┬───────────────────────┘
                                 │
                                 ▼
          ┌──────────────────────────────────────────────┐
  S530    │  determining cause of wrinkle formation      │
          │  based on first wrinkle information and       │
          │  second wrinkle information                   │
          └──────────────────────┬───────────────────────┘
                                 │
                                 ▼
                         ┌──────────────┐
                         │     END      │
                         └──────────────┘
```

FIG. 6

## INTERNATIONAL SEARCH REPORT

| | International application No. |
|---|---|
| | **PCT/KR2023/008955** |

**A.    CLASSIFICATION OF SUBJECT MATTER**

**A61B 5/00**(2006.01)i

According to International Patent Classification (IPC) or to both national classification and IPC

**B.    FIELDS SEARCHED**

Minimum documentation searched (classification system followed by classification symbols)

A61B 5/00(2006.01); A61B 5/103(2006.01); G06Q 50/22(2012.01); G16H 50/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다파장(multi-wavelength), 피부(skin), 분석(analysis), 추천 (recommendation), 화장품(cosmetics), 멜라닌(melanin), 다크서클(dark circle), 주름(wrinkle)

**C.    DOCUMENTS CONSIDERED TO BE RELEVANT**

| Category* | Citation of document, with indication, where appropriate, of the relevant passages | Relevant to claim No. |
|---|---|---|
| Y | KR 10-2021-0069495 A (SAMSUNG ELECTRONICS CO., LTD.) 11 June 2021 (2021-06-11)<br>See paragraph [0046]; and claim 1. | 1-5 |
| Y | KR 10-2014-0112046 A (CHANEL PARFUMS BEAUTE) 22 September 2014 (2014-09-22)<br>See paragraphs [0027] and [0029]; and claim 1. | 1-5 |
| Y | KR 10-2015-0080280 A (AMOREPACIFIC CORPORATION) 09 July 2015 (2015-07-09)<br>See paragraphs [0029], [0056]-[0057], [0060] and [0070]; and claim 8. | 1-5 |
| Y | KR 10-2022-0009735 A (COCORY COLOR RESEARCH INSTITUTE, INC.) 25 January 2022 (2022-01-25)<br>See paragraph [0033]; and claim 1. | 1-5 |
| A | KR 10-2020-0142938 A (LULULAB INC.) 23 December 2020 (2020-12-23)<br>See paragraphs [0027]-[0056]; and figures 1-2. | 1-5 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 October 2023** | **04 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

EP 4 548 833 A1

**INTERNATIONAL SEARCH REPORT**

| International application No. |
|---|
| **PCT/KR2023/008955** |

**C. DOCUMENTS CONSIDERED TO BE RELEVANT**

| Category* | Citation of document, with indication, where appropriate, of the relevant passages | Relevant to claim No. |
|---|---|---|
| PX | KR 10-2450422 B1 (LULULAB INC.) 06 October 2022 (2022-10-06)<br>See paragraphs [0032]-[0075]; claims 1-2 and 4-5; and figures 1 and 3.<br>※ This document is a published earlier application that serves as a basis for claiming priority of the present international application. | 1-5 |

Form PCT/ISA/210 (second sheet) (July 2022)

17

**INTERNATIONAL SEARCH REPORT**
Information on patent family members

| | International application No. |
|---|---|
| | **PCT/KR2023/008955** |

| Patent document cited in search report | | | Publication date (day/month/year) | Patent family member(s) | | | Publication date (day/month/year) |
|---|---|---|---|---|---|---|---|
| KR | 10-2021-0069495 | A | 11 June 2021 | WO | 2021-112447 | A1 | 10 June 2021 |
| KR | 10-2014-0112046 | A | 22 September 2014 | FR | 2984721 | A1 | 28 June 2013 |
| | | | | FR | 2984721 | B1 | 28 June 2013 |
| | | | | JP | 2015-500722 | A | 08 January 2015 |
| | | | | WO | 2013-098512 | A1 | 04 July 2013 |
| KR | 10-2015-0080280 | A | 09 July 2015 | KR | 10-2175251 | B1 | 09 November 2020 |
| KR | 10-2022-0009735 | A | 25 January 2022 | KR | 10-2396376 | B1 | 10 May 2022 |
| | | | | US | 2023-0189964 | A1 | 22 June 2023 |
| | | | | WO | 2022-015104 | A1 | 20 January 2022 |
| KR | 10-2020-0142938 | A | 23 December 2020 | US | 2022-0344044 | A1 | 27 October 2022 |
| | | | | WO | 2020-251217 | A1 | 17 December 2020 |
| KR | 10-2450422 | B1 | 06 October 2022 | | None | | |

Form PCT/ISA/210 (patent family annex) (July 2022)